# EUROPEAN PATENT APPLICATION

(11) **EP 1 283 271 A1**
(43) Date of publication of application: **12.02.2003**
(21) Application number: 01119370.3
(22) Date of filing: 10.08.2001
(51) Int. Cl.: C12Q 1/68, G01N 33/50

(54) **Method of screening compounds using nematode worms**

(71) Applicant: Elegene AG, 82152 Planegg (DE)
(72) Inventor: Baumeister, Ralf, Prof. Dr., 82194 Gröbenzell (DE); Höner, Marius, Dr., 81247 München (DE); Nguyen, Tri, Dr., 81377 München (DE); Tovar, Karlheinz, Dr., 82239 Alling (DE); Weidner, Gerhard, Dr., 82131 Gauting (DE)
(74) Representative: Stoppkotte, Cornelia

(57) **Abstract**

The present invention relates to a method of screening compounds for pharmacological activities using nematode worms. In particular, the present invention relates to a method of screening compounds for activity in treating and/or preventing muscular dystrophy using a nematode worm having a loss of function of the dystrophin *(dys-1)* gene which is preferably caused by mutation or RNA interference. The inventive screening method is conducted under such conditions that the nematode worm shows a phenotype of at least nearly complete paralysis. This phenotype is especially suited for screening tests.

## Description

The present invention relates to a method of screening compounds for pharmacological activities using nematode worms. In particular, the present invention relates to a method of screening compounds for activity in treating and/or preventing muscular dystrophy using a nematode worm having a loss of function of the dystrophin (*dys-1*) gene.

Duchenne muscular dystrophy (DMD) is a X-linked recessive disease that affects 1/3500 male births. DMD patients suffer progressive skeletal muscle degeneration; they loose the ability to walk by the age of 12. Furthermore, these DMD patients also suffer from lung and cardiac defects resulting in early death by the age of 20. The human dystrophin gene was identified and cloned by Koenig et al. (1988). Subsequently, dystrophin orthologues were identified in other organisms such as mouse (Sicinski et al., 1989), *Drosophila* (Greener and Roberts, 2000) and *Caenorhabditis elegans* (Bessou et al., 1988). Structurally, the dystrophin protein is evolutionary conserved and comprised of an actinin-type actin binding domain at the N-terminus, a series of beta-spectrin like domains in the middle region, and a WW domain along with a coiled-coil domain at the C-terminus (Koenig et al., 1988). Dystrophin is part of an elaborate protein complex linking the inner cytoskeleton with the extracellular matrix providing structural support to the sarcolemma membrane and resistance to injury from constant mechanical forces associated with muscle usage and skeletal muscle growth. In the absence of dystrophin, muscle cells become unstable. Thus, this leads to muscle atrophy and eventually muscle necrosis. For the time being, there is no cure for DMD.

Since 1974, the use of corticosteroids to treat DMD patients was subjected to numerous studies. Clinical studies conducted in the 70's, 80's and early 90's have shown that DMD patients treated with 0.75 mg/kg/day of prednisone improved muscle strength and function compared to placebo. DMD patients treated with prednisone have been shown to have positive effects such as muscle mass increase. In all studies, these patients showed a generation of increased muscular force allowing the patients to climb stairs faster, to stand up quicker, and to have an increase in leg and arm function. However, the side-effects are often unpreventable; DMD patients treated with prednisone suffer cushinggoid, elevation of blood pressure, up to 40% reduction in height gain, cataracts, skin rash, and significant weight gain. The molecular mechanisms of prednisone in DMD patients are still unclear. Biochemical studies have shown that DMD patients treated with prednisone showed an increase in urinary creatinine excretion suggesting that there is an elevation in muscle mass. Moreover, treated patients also showed a decrease in 3-methylhistidine indicating a decrease in muscle break down. With the current drug treatment, it allows DMD patients to live an average of 3 years longer, but long-term effects of prednisone are still unknown.

The dystrophin gene in *C. elegans* was identified and cloned, and the protein sequence has been published (Bessou et al. (1998), which is incorporated herein by reference). Furthermore, the protein sequence of 3674 amino acids has been submitted to the EMBL database with the accession number AJO12469. Bessou et al. (1998) also describe several loss of function mutations: *cx35, cx18, cx26, cx40* and *ad538.* The *dys-1*(*cx18*) mutant, for example, has a G to A point mutation, which leads to a premature stop codon at amino acid 2721 resulting in a truncated protein. When *cx18* mutant animals were grown on agar plates at 20 °C, they exhibited hyperactivity in backward movement and bending of the head (Bessou et al., 1998). Furthermore, Bessou et al. (1998) showed that *C. elegans dys-1* mutants could be rescued with the human dystrophin gene. This suggests that the human and *C. elegans* dystrophin are functionally conserved. Thus, this provides the possibility that investigators can use *C. elegans* as a model system to study muscular dystrophy.

Based on the above described state of the art, it is the object of the present invention to provide a new method for screening compounds for potential pharmaceutical activity using nematode worms, in particular to provide a screening method for identifying compounds which can be used to treat and/or prevent muscular dystrophy, in particular Duchenne muscular dystrophy.

The present inventors surprisingly found that the *C. elegans* muscular dystrophy loss of function mutants, in particular *dys-1(cx18),* are paralyzed and unable to develop under certain conditions, in particular in liquid culture at a temperature of about 21 to 27 °C, whereas wild type shows a normal development under these conditions. This phenotype of paralysis is particularly well suited for screening tests. Furthermore, the inventors found that prednisone can be used to reverse paralysis and development defects. Thus, the inventors concluded that nematode worms, in particular *C. elegans,* are an excellent model system for high-throughput screening (HTS) to identify novel compounds that eventually can be used for therapeutic drugs for DMD.

Thus, the object of the present invention is solved by a screening method comprising the following steps:
(a) Keeping a nematode worm having a loss of function of the *dys-1* gene under such conditions that it shows a phenotype of at least nearly complete paralysis;
(b) Exposing the worm to a compound to be tested; and
(c) Measuring and/or observing any change in the phenotype and/or behaviour of the nematode worm.

In this method, the nematode worm preferably belongs to the genus *Caenorhabditis* and, most preferably, is *Caenorhabditis elegans (C. elegans).*

With regard to the loss of function of the *dys-1* gene, this loss of function can be caused by one or more genetic mutations (such as for example *cx35, cx18, cx26, cx40,* and *ad538* with regard to the *C. elegans dys-1* gene) and/or RNA interference which technique is described in detail in Fire et al., 1998. With regard to the above mentioned mutations, the *cx18* mutation is preferred.

In the inventive method, the worm is preferably kept in liquid culture, and especially preferred is M9 medium [6g Na₂HPO₄, 3g KH₂PO₄, 5g NaCl, 0.25g MgSO₄·7H₂O per litre H₂O] (Epstein and Shakes, 1995).

The growth temperature of the liquid culture has to be selected such that the nematode worms used show a phenotype of nearly complete or complete paralysis. With the C. *elegans* mutant *dys-1*(*cx18*), this phenotype, which is especially well suited for screening tests because changes in the phenotype can easily be detected, was found in a temperature range of the liquid culture of 21 to 27 °C, preferably about 24 to 26 °C, and most preferably at about 25 °C.

The inventive method is particularly useful to identify compounds which can be used to treat and/or prevent muscular dystrophy, in particular Duchenne muscular dystrophy. The inventive method can favourably be conducted as a high-throughput screening method which leads to a high efficiency of the screening procedure.

Novel compounds for treating muscular dystrophy, in particular Duchenne muscular dystrophy, identified by the inventive method are also within the scope of the present invention.

The present invention will now be illustrated in more detail by the following example.

### EXAMPLE

### Strains:

*C. elegans* wild type (N2) and *dys-1(cx18)* mutants used in this study were obtained from the *Caenorhabditis* Genetics Center (University of Minnesota, U.S.A.). Animals were cultured as previous described by Brenner (1974). The animals were maintained at 20 °C.

### Egg Preparation:

All experiments were done in parallel using *dys-1* mutants and wild type animals. N2 and *dys-1(cx18)* animals were grown on 9 cm seeded plates with either *E. coli* strain OP50 or HB 101. Both strains were obtained from the *Caenorhabditis* Genetics Center. For each experiment, 10 plates of each strain were used. The worm plates were washed with 0.2% Tween®-20 solution (Tween®-20 solution) and collected in 15 ml conical falcon tubes followed by 1 minute centrifugation at 1000 rpm at 20 °C in a table top centrifuge. This process was repeated once more. The supernatant was discarded and 10 ml of solution containing: 0.5% NaOCl; 0.25 mM KOH (bleach solution) were added and the tubes were shaken for 8-10 minutes to dissolve worm carcass leaving only eggs behind. The eggs were then centrifuged for 1 minute at 1000 rpm and the supernatant was discarded. The eggs were washed 6 more times with Tween®-20 solution then filtered through a 40 µm cell strainer (Falcon Nylon) allowing only the eggs to pass through leaving everything else behind. The eggs were washed one more time before resuspending in 6 ml of M9 buffer as described above (Epstein and Shakes, 1995). The eggs were placed on a rotator overnight (18-19 hours) at 20 °C. The next day, the concentration of the worms was determined by counting 10 aliquots of 10 µl manually. An average was then determined.

### Bacteria Preparation:

A single *E. coli* colony HB 101 was grown in LB medium [10 g tryptone, 5 g yeast extract, 5 g NaCl per litre H₂O] (Ausubel et al., 1999) over night at 37 °C on a shaker. 100 ml of the culture was poured into a 50 ml Falcon-tube and centrifuged for 3 minutes at 3500 rpm. The pellet was washed three times in M9 buffer and then resuspended in 10 ml of M9. The concentration of bacteria was measured.

### Drug Screen

All assays were done in 96 or in 384 well plates. Each well contained approximately 50-200 larvae (L1) along with 0.25 to 0.5 OD of bacteria. In addition, a final concentration of 20 µg/ml of chloramphenicol dissolved in M9 was used to inhibit bacterial cell division for the imaging reasons. Prednisone (Sigma-Alrich Chemical, St. Louis) dissolved in DMSO was added as a drug. During the studies, six different final drug concentrations were used: 5 µM, 10 µM, 25 µM, 100 µM, 200 µM and 250 µM. Appropriate controls were done at the same time. The worms along with the drug were incubated at 25 °C in a moist chamber for at least 22-30 hours. After 26 hours at 25 °C, most of the animals reached larval stage 4 (L4). Scoring was done between 26 and 28 hours after the drug addition. Scoring was done manually by taking an aliquot each time to count. Animals were scored based solely on their motility phenotype: either the animals were completely paralyzed or any sort of movement was counted as "movement" using a dissecting microscope.

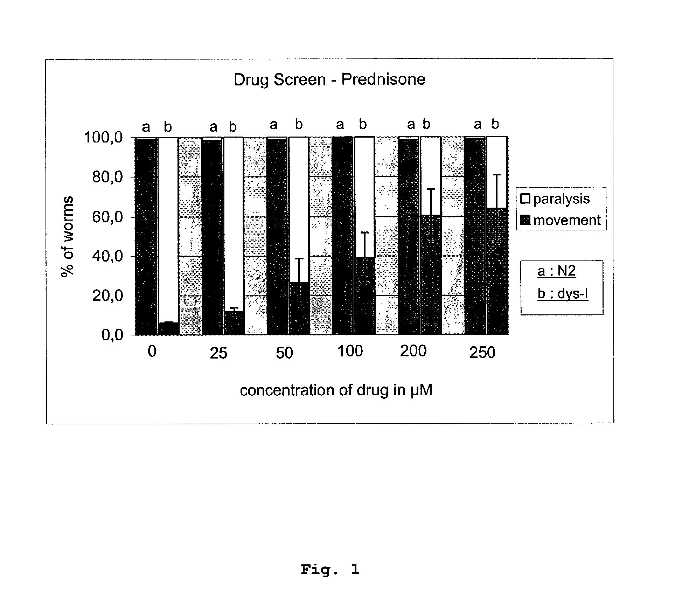

The results obtained in the above assays are shown below in Table 1 as well as in Fig. 1.

**Table 1**

| Drug Concentration: 0 | | | 25µM | | 50µM | | 100µM | | 200µM | | 250µM | |
|---|---|---|---|---|---|---|---|---|---|---|---|---|
| Strain: | N2 | dys-1 | N2 | dys-1 | N2 | dys-1 | N2 | dys-1 | N2 | dys-1 | N2 | dys-1 |
| Movement %: | 98.9 | 6.0 | 98.5 | 11.8 | 98.6 | 26.5 | 99.2 | 39.1 | 98.5 | 60.5 | 99.1 | 64.1 |
| Paralysis %: | 1.1 | 94.0 | 1.5 | 88.2 | 1.4 | 73.5 | 0.8 | 60.9 | 1.5 | 39.5 | 0.9 | 35.9 |
| Total number(n) | 1386 | 1012 | 1361 | 1060 | 1308 | 1176 | 1394 | 1361 | 1506 | 1521 | 1106 | 1077 |

The above described experiment showed that C. elegans *dys-1*(*cx18*) mutant worms growing in M9 liquid culture at 25 °C showed 95% paralysis compared to the wild type (N2) worms. This paralysis could be reversed in the presence of prednisone. It appeared that the reversion of paralysis was drug concentration dependent. At the higher concentration of 250 µM of predisone, the *dys-1(cx18)* mutant animals showed over 50% improvement. M9 medium (without prednisone; "0" in Table 1 and Fig. 1) was used as a negative control.

### References

Ausubel et al. (1999). Short Protocols in Molecular Biology, 4th Edition, Wiley Publisher, New York.
Bessou et al. (1998) Mutations in Caenorhabditis elegans dystrophin-like gene dys-1 lead to hyperactivity and suggest a link with cholinergic transmission. Neurogenetics 2: 61-72.
Brenner, S. (1974) The genetics of *Caenorhabditis* elegans. Genetics 77: 71-94.
Epstein and Shakes (1995) *Caenorhabditis elegans*: Modern Biological Analysis of an Organism. Volume 48, Academic Press, New York.
Fire et al. (1998) Potent and specific genetic interference by double-stranded RNA in Caenorhabditis elegans. Nature 391: 806. Greener and Roberts (2000) Conservation of components of the Dystrophin complex in *Drosophila.* FEBS Letters 482: 13-18. Koenig et al. (1988) Complete cloning of the Duchenne muscular dystrophy (DMD) cDNA and preliminary genomic organization of the DMD gene in normal and affected individuals. *Cell* 50: 509-17.
Sicinski et al. (1989) The molecular basis of muscular dystrophy in the mdx mouse: a point mutation. Science 244: 1578-1580.

## Claims

1. A method of screening compounds for potential pharmacological activity using a nematode worm, which method comprises the steps of:
(a) Keeping a nematode worm having a loss of function of the *dys-1* gene under such conditions that it shows a phenotype of at least nearly complete paralysis;
(b) Exposing the worm to a compound to be tested; and
(c) Measuring and/or observing any change in the phenotype and/or behaviour of the nematode worm.

2. A method according to claim 1, wherein the nematode worm belongs to the genus *Caenorhabditis.*

3. A method according to claim 2, wherein the nematode worm is *Caenorhabditis elegans (C. elegans).*

4. A method according to any of claims 1 to 3, wherein the loss of function of the *dys-1* gene is caused by mutation and/or by RNA interference.

5. A method according to claim 4, wherein the mutation is the *cx35, cx18, cx26, cx40* or *ad538* mutation, preferably the *cx18* mutation, of the *C. elegans dys-1* gene.

6. A method according to any of claims 1 to 5, wherein the nematode worm is kept in liquid culture, preferably in M9 medium.

7. A method according to claim 6, wherein the temperature of the liquid culture is in the range of about 21 to 27°C, preferably about 24 to 26°C, and most preferably is about 25°C.

8. A method according to any of claims 1 to 7, wherein the pharmacological activities of the screened compounds reside in the ability to treat and/or prevent muscular dystrophy, in particular Duchenne muscular dystrophy.

9. A method according to any of claims 1 to 8, wherein the method is a high-throughput screening method.

10. Use of a nematode worm having a loss of function of the *dys-1* gene for screening compounds of possible utility in treating and/or preventing muscular dystrophy, in particular Duchenne muscular dystrophy.

11. Use according to claim 10, wherein the loss of function is caused by mutation and/or by RNA interference.

12. Use according to claim 11, wherein the mutation is the *cx35, cx18, cx26, cx40* or *ad538* mutation, preferably the *cx18* mutation, of the *C. elegans dys-1* gene.

13. Novel compounds for treating muscular dystrophy, in particular Duchenne muscular dystrophy, identified by a method according to any of claims 1 to 9.
